# EUROPÄISCHE PATENTANMELDUNG

(11) **EP 2 014 260 A1**
(43) Veröffentlichungstag der Anmeldung: **14.01.2009**
(21) Anmeldenummer: 07075729.9
(22) Anmeldetag: 23.08.2007
(51) Int. Cl.: A61F 2/36, A61F 2/40

(54) **Mehrteiliges oder modulares orthopädisches und traumatologisches Implantat**

(30) Priorität: 11.07.2007 DE 102007033016
(71) Anmelder: AAP Implantate AG, 12099 Berlin (DE)
(72) Erfinder: Fischer, Hans-J., Dr.-Ing., 12277 Berlin (DE)
(74) Vertreter: Meissner, Peter E.

(57) **Zusammenfassung**

Die Erfindung betrifft ein mehrteiliges oder modulares orthopädisches und traumatologisches Implantat, dessen Verbindungen fügbar und lösbar sind, wobei die eine Komponente mit einer bohrungsförmigen Struktur und die Gegenkomponente mit einer zapfenförmigen Struktur (1) versehen ist und durch diese neben einer formschlüssigen Verbindung auch eine definierte Winkelposition festlegbar ist, und wobei die zapfenförmige Struktur auf die bohrungsförmige Struktur abgestimmte Kontaktbereiche und kontaktfreie Bereich aufweist, die zu einer Spannungsoptimierung und der Reduzierung von Mikrobewegungen in der Verbindung führen.

## Beschreibung

Die Erfindung betrifft ein mehrteiliges oder modulares orthopädisches und traumatologisches Implantat.

Die modulare Verbindung zwischen zwei oder mehr Komponenten bietet die Möglichkeit einer intra-operativen Anpassung des Implantates. z. B. können hierdurch unterschiedliche Halsstückgeometrien durch die intra-operative Anpassung des CCD-Winkels, des Offsets und der Antetorsion erzielt werden. Dadurch kann eine Rekonstruktion der anatomischen Gelenkfunktion unter Berücksichtigung des Rotationszentrums und der Weichteilsituation erreicht werden. Durch die Variabilität von Prothesenhals und -kopf können auch der freie Bewegungsumfang und die Beinlänge besser eingestellt werden. Der Prothesenschaft kann so mit unterschiedlichen Halsstücken die verschiedenen CCD- Winkeln, korrespondierende Offsetvarianten sowie Antetorsionswinkeln beinhalten, kombiniert werden. Als Verbindungselemente werden meistens Konusverbindungen mit rundem oder ovalem Querschnitt und kleinem Kegelwinkel eingesetzt. Durch eine integrierte oder zusätzliche Rotationssicherung wird ein Verdrehen der Verbindung ausgeschlossen.

Im Gegensatz zu den Vorteilen der Modularität beinhaltet jede zusätzliche Implantatsschnittstelle klinische Risiken wie Partikelgenerierung durch Reibverschleiß (so genanntes "fretting") an den Grenzflächen der Verbindungsstelle, ein erhöhtes Risiko von Ermüdungsbrüchen unter dynamischer Belastung sowie die Möglichkeit einer post-operativen Separation der Hals/Schaft-Verbindung.

Besonders aus den produktionstechnisch bedingten Toleranzen zusammen mit der ungünstigen Krafteinleitung in die Konusverbindung, entstehen im oberen Konusabschnitt medial und im unteren Bereich der Konusverbindung lateral sehr ungünstige hohe Kontaktspannung. In Kombination mit Mikrobewegungen durch die unvermeidlichen Formabweichungen und Toleranzen, sind hier bei den bekannten Lösungen ein erhöhter Schwingungsreibverschleißes und ein ungünstiges Tragverhalten zu erwarten.

Ein Beispiel für ein Implantat ist eine Endoprothese für ein künstliches Hüftgelenk.

Diese kann dreiteilig aufgebaut sein und besteht dann aus einem Kopfteil, einem Schaft und einem beide verbindenden Übergangsstück oder Zapfenelement.

Dieses Übergangsstück weist eine distale zapfenförmige Struktur auf, die in eine entsprechende Bohrung am proximalen Ende des Schaftes einsetzbar ist, während eine proximale zapfenförmige Struktur mit dem Kopfteil verbindbar ist.

Unterschiedliche Ausbildungen dieses Übergangsstückes sind aus der EP 797 964 A1, der DE 11 2004 001 893 T5 und der DE 199 40 348 A1 bekannt.

Bei der letzt genannten Veröffentlichung weist die distale zapfenförmige Struktur ein Vielkeilprofil auf, so dass ein rotationssicheres Raster zur optimalen Einstellung des Antetorsions - und CCD-Winkels ermöglicht wird.

Vergleichbare Verbindungen eines eine zapfenförmige Struktur tragenden Teiles und eines zugeordneten Teiles mit einer Bohrung in die diese zapfenförmige Struktur einsetzbar ist, sind auch bei modularen Implantatssystemen üblich, die im Bereich Hüft-, Schulter- oder Knierevisonen eingesetzt werden .

Die Zielsetzung dieser Erfindung ist es biomechanischen Vorteile solcher modularen Verbindungen zu Nutzen und gleichzeitig die daraus resultierenden Risiken zu minimieren.

### Hier sind besonders die Gefahren wie Ermüdungsbrüche sowie der

Schwingungsreibverschleiß von modularen Halsverbindungen zu reduzieren. Diese entstehen in erster Linie durch die biomechanisch bedingte Einleitung von Wechsellasten in die bekannten Konusverbindungen.

Gelöst wird diese Aufgabe erfindungsgemäß mit einem mehrteiligen oder modularen orthopädischen und traumatologischen Implantat, dessen Verbindungen fügbar und lösbar sind, wobei die eine Komponente mit einer bohrungsförmigen Struktur und die Gegenkomponente mit einer zapfenförmigen Struktur versehen ist und durch diese neben einer formschlüssigen Verbindung auch eine definierte Winkelposition festlegbar ist, und wobei die zapfenförmige Struktur auf die bohrungsförmige Struktur abgestimmte Kontaktbereiche und kontaktfreie Bereiche aufweist, die zu einer Spannungsoptimierung und der Reduzierung von Mikrobewegungen in der Verbindung führen.

Durch die besondere Form des Verbindungszapfens zur korrespondierenden Konusform wird der Einfluss von Formabweichungen und Toleranzen deutlich verringert. Zusätzlich erlaubt die elastische Anpassung des Verbindungszapfens

Bei den bekannten Verbindungen wurde beispielsweise die zapfenförmige Struktur als Konus ausgebildet und die zugeordnete Bohrungsstruktur besaß eine entsprechende konische Innenausbildung.

Wenn die Achse der Krafteinleitung mit der Achse der zapfenförmigen Struktur und der der Bohrungsstruktur übereinstimmte, ergaben sich keine Probleme.

Wurde allerdings ein winkliges Übergangsstück eingesetzt um das Kopfteil mit dem Schaftteil einer Endoprothese zu verbinden, so erfolgte die Krafteinleitung unter einem Winkel zur Achse der Bohrung. Damit war bei einer gewissen Toleranz zwischen Konus und Bohrung ein quasi Kippmoment nicht zu vermeiden und dies hatte zur Folge, dass die Flächenpressung zwischen den beiden lösbar verbundenen Komponenten ungleich war. Wenn dies nun vorher bei der Berechnung von Zapfenstruktur bzw. Bohrung berücksichtigt wird, dann kann die Flächenpressung durch entsprechende Gestaltung der sich berührenden Flächen vergleichmäßigt werden.

Dies läßt sich nach einem Ausführungsbeispiel der Erfindung dadurch erreichen, dass die zapfenförmige Struktur eine dessen Elastizität verändernde, z. B. erhöhende Innenbohrung oder Innenkontur aufweist, die zum distalen Ende der zapfenförmigen Struktur hin offen ist.

Nach einer weiteren vorzugsweisen Ausgestaltung ist am Umfang einer der Enden der zapfenförmige Struktur ein Raster vorgesehen, das mit einem Raster am oberen Rand der bohrungsförmigen Struktur der Gegenkomponente zur Einstellung der definierten Winkelposition in Eingriff bringbar ist.

In weiterer Ausgestaltung der Erfindung handelt es sich um ein Implantat in Form eines modularer Implantates, mit einer distalen Komponente und einem zur Längsachse gewinkeltem Übergangsstück, dessen proximale Seite mit einem Hüftkopf und dessen distales Ende mit einer Schaftkomponete lösbar verbunden ist, wobei die distale Komponente mit Preßsitz in einer Bohrung der Schaftkomponente mit definierter Winkelposition bezüglich des Schaftes einsetzbar ist, und die distale Seite des Übergangsstückes - bezogen auf die lichte Weite der Bohrung im Schaft - in Längsrichtung bereichsweise oder punktuell ein Übermaß aufweist.

Dabei sollte das Übermaß an der distalen Seite im Bereich des Außenwinkels des gewinkelten Übergangsstückes vorgesehen sein.

Vorzugsweise ist am Umfang des proximalen Endes der zapfenförmigen Struktur ein Raster vorgesehen, das mit einem Raster am oberen Rand der Bohrung im Schaft zur Einstellung der definierten Winkelposition in Eingriff bringbar ist.

Die Erfindung soll nachfolgend unter Bezug auf die Zeichnungen an einem Ausführungsbeispiel erläutert werden.

Dabei zeigt:
- Fig. 1: ein Zapfenelement für eine Endoprothese,
- Fig. 2: eine Detailansicht und
- Fig. 3: eine Aufsicht.

Bei den bisherigen Zapfenelementen ist der Konus so ausgebildet, dass er sich zum freien Ende hin gleichmäßig verringert.

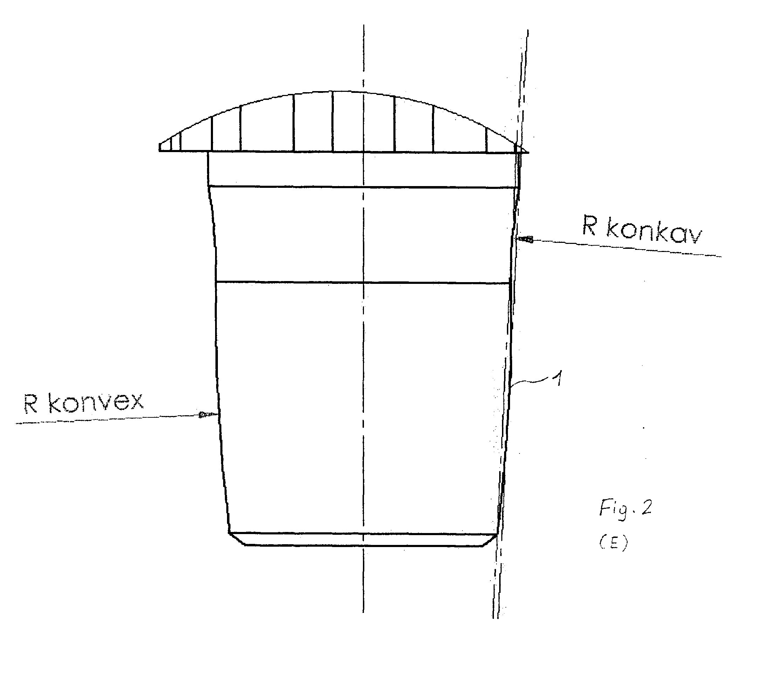

Aus der Detailansicht in Figur 2 ist nun zu erkennen, dass bei der erfindungsgemäßen Ausbildung der zapfenförmigen Struktur 1 ein oberer Teilbereich konkav und ein unterer - dem freien Ende zugewandter Bereich - konvex gestaltet ist. Damit ergeben sich unterschiedliche Anlageflächen an der hier nicht dargestellten bohrungsförmigen Struktur, beispielsweise im proximalen Ende eines Schaftes. Hierdurch ergeben sich wiederum optimierte Flächenpressungen.

Bei der zeichnerischen Darstellung wurde vereinfacht von einer umlaufenden Außendurchmesserveränderung ausgegangen. Es ist klar, dass zu einer Optimierung natürlich auch die besondere Gestaltung einzelner Flächenbereiche gehört, wenn diese - beispielsweise bedingt durch eine winklige Krafteinleitung - besonderen Flächenpressungen unterliegen. Diese veränderten Bereiche können sowohl in Längsrichtung der zapfenförmigen Struktur wie auch über den Umfang verteilt ausgebildet werden.

Wie vorstehend bereits erwähnt, kann eine Vergleichmäßigung der Flächenpressung auch durch die Anbringung definierter Bohrungen in der zapfenförmigen Struktur erreicht werden, die dann die Verformung oder die Elastizität der zapfenförmigen Struktur in der gewünschten Weise beeinflusst.

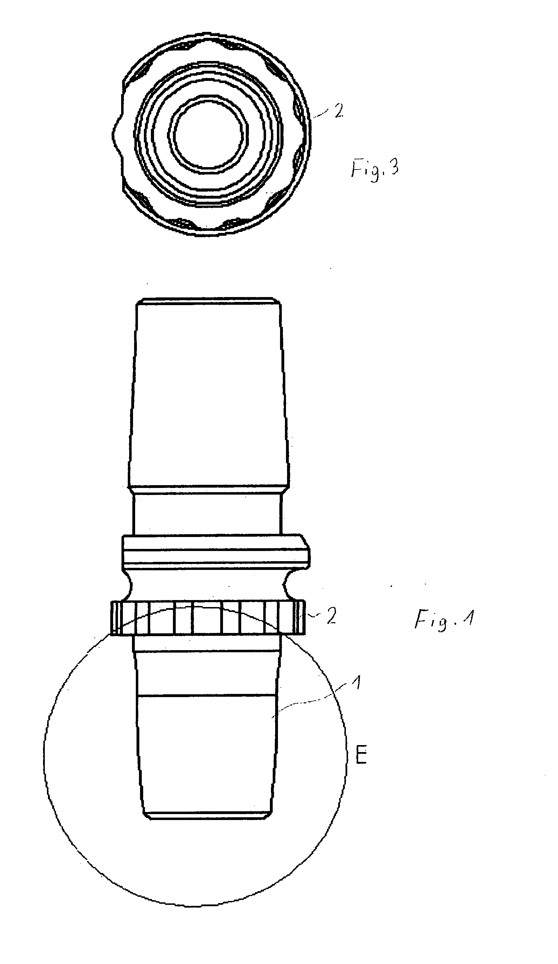

Die Figur 3 zeigt in der Aufsicht, dass am Umfang der zapfenförmige Struktur 1 ein Raster 2 vorgesehen, das mit einem Raster am oberen Rand der bohrungsförmigen Struktur der Gegenkomponente zur Einstellung der definierten Winkelposition in Eingriff bringbar ist.

## Patentansprüche

1. Mehrteiliges oder modulares orthopädisches und traumatologisches Implantat, dessen Verbindungen fügbar und lösbar sind, wobei die eine Komponente mit einer bohrungsförmigen Struktur und die Gegenkomponente mit einer zapfenförmigen Struktur versehen ist und durch diese neben einer formschlüssigen Verbindung auch eine definierte Winkelposition festlegbar ist, und wobei die zapfenförmige Struktur auf die bohrungsförmige Struktur abgestimmte Kontaktbereiche und kontaktfreie Bereiche aufweist, die zu einer Spannungsoptimierung und der Reduzierung von Mikrobewegungen in der Verbindung führen.

2. Implantat nach Anspruch 1,
**dadurch gekennzeichnet,**
**dass** die zapfenförmige Struktur der Verbindung eine dessen Elastizität erhöhende Innenbohrung / Innenkontur aufweist, die zum auslaufende Ende dieser zapfenförmigen Struktur hin offen ist.

3. Implantat nach einem der vorstehenden Ansprüche,
**dadurch gekennzeichnet,**
**dass** am Umfang einer der Enden der zapfenförmige Struktur ein Raster vorgesehen ist, das mit einem Raster am oberen Rand der bohrungsförmigen Struktur der Gegenkomponente zur Einstellung der definierten Winkelposition in Eingriff bringbar ist.

4. Implantat nach einem der vorstehenden Ansprüche, in Form modularer Implantate, mit einer distalen Komponente und einem zur Längsachse gewinkeltem Übergangsstück, dessen proximale Seite mit einem Hüftkopf und dessen distales Ende mit einer Schaftkomponete lösbar verbunden ist, wobei die distale Komponente mit Preßsitz in einer Bohrung der Schaftkomponente mit definierter Winkelposition bezüglich des Schaftes einsetzbar ist, und die distale Seite des Übergangsstückes - bezogen auf die lichte Weite der Bohrung im Schaft - in Längsrichtung bereichsweise oder punktuell ein Übermaß aufweist.

5. Implantat nach Anspruch 4,
**dadurch gekennzeichnet,**
**dass** das Übermaß an der distalen Seite im Bereich des Außenwinkels des gewinkelten Übergangsstückes vorgesehen ist.

6. Implantat nach einem der vorstehenden Ansprüche,
**dadurch gekennzeichnet,**
**dass** am Umfang des proximalen Endes der zapfenförmigen Komponente ein Raster vorgesehen ist, das mit einem Raster am oberen Rand der Bohrung im Schaft zur Einstellung der definierten Winkelposition in Eingriff bringbar ist.
